(19) Europäisches Patentamt
European Patent Office
Office européen des brevets

(11) Publication number: 0 330 374 A2

(12) **EUROPEAN PATENT APPLICATION**

(21) Application number: 89301500.8

(51) Int. Cl.4: C12Q 1/36 , C07K 5/00

(22) Date of filing: 16.02.89

(30) Priority: 22.02.88 US 158875

(43) Date of publication of application: 30.08.89 Bulletin 89/35

(84) Designated Contracting States: AT BE CH DE ES FR GB GR IT LI LU NL SE

(71) Applicant: Smith, Robert Eugene
574 Escondido Circle
Livermore California 94550(US)

Applicant: Brown, Elvin E.
7100 San Ramon Boulevard 164
Dublin California 94568(US)

(72) Inventor: Smith, Robert Eugene
574 Escondido Circle
Livermore California 94550(US)
Inventor: Brown, Elvin E.
7100 San Ramon Boulevard 164
Dublin California 94568(US)

(74) Representative: Atkinson, Peter Birch et al
Marks & Clerk Suite 301 Sunlight House
Quay Street
Manchester M3 3JY(GB)

# (54) Method for the colorimetric determination of DPP-IV using cinnamaldehyde.

(57) A method for the colorimetric determination of DPP-IV comprising adding an unknown to a substrate having a leaving group cleavable by the DPP-IV enzyme, incubating the mixture of the unknown and substrate, adding cinnamaldehyde to the unknown to combine with the leaving group to form a color change, and evaluating the color to determine the presence of DPP-IV. The preferred substrate is L-Alanyl-L-Proline-4-Methoxy-2-Naphthylamine.

EP 0 330 374 A2

Xerox Copy Centre

# METHOD FOR THE COLORIMETRIC DETERMINATION OF DPP-IV USING CINNAMALDEHYDE

## Background of the Invention

### Field of the Invention:

The present invention relates to the field of assay methods and reagents for determination of enzymes, and particularly to the assaying of unknowns for the presence of dipeptidyl-(amino)-peptidase-IV.

### Description of the Prior Art:

Dipeptidyl-(amino)-peptidase-IV is a post-proline exopeptidase found in various animal tissues. This enzyme is identified by a variety of equivalent names, including glycyl-proline-naphthyl amidase, glycyl-proline-4-methoxy-beta-napthylamide-peptidase and DPP-IV. DPP-IV was first described as in intra-cellular enzyme that hydrolyzed gly-pro-naphthylamide in commercial preparations of Porcine Kidney Acylase and in rat liver extracts. Hopsu-Havu, V. K. & Glenner, G. G., "A New Di-Peptide-Naphthyl-Amidase Hydrolizing Glycyl-Prolyl-Beta-Naphthlyamide"; Histochemie, 7:197-201 (1966).

Studies have been conducted for the presence and role of DPP-IV in various animal tissues. David et al., Proteins of the Kidney Microvillar Membrane, Biochem. J. 179, pp. 379-395 (1979); Elovson, Biogenesis of Plasma Membrane Glycoproteins, J. Biological Chemistry, vol. 255, no. 12, pp. 5807-5815 (1980); Kreil et al., Stepwise Cleavage of the Pro Part of Promelittin by Dipeptidylpeptidase IV, Eur. J. Biochem. 111, pp. 49-58 (1980). It has been established that most vertebra tissues contain the enzyme and it has been purified from a number of different animal tissues. McDonald, J.K., Callahan, P. X., Ellis, S. & Smith, R. E., "Polypeptide Degradation by Di-Peptidal Aminopeptidase I (Cathepsin C) and Related Peptidases". In: Tissue Proteinases (Barrett A. J. & Dingle, J. T., eds.), pp. 69-107 North-Holland Publishing Co., Amsterdam, 1971; McDonald, J. K., and Barrett A. J., "Entry 13.04 Dipeptidal Peptidase IV" in Mammalian Proteinases: A Glossary and Bibliography, Vol. 2, pp. 132-149, Academic Press, London, 1986; Puschel, G., Mentlein, R. & Heymann, E., "Isolation and Characterization of Dipeptidal Peptidase IV from Human Placenta", Euro. J. Biochem., 126:359-365, 1982.

DPP-IV is located primarily in cell membranes, and most commonly on the outer cell surface of the plasma membrane. The enzyme has been found in various tissues and body fluids including the liver, kidney, salivary glands, serum and urine. The enzyme activity in human serum is increased in patients with hepatic disorders, and decreased in patients with cancer, acute lymphocytic leukemia, Hodgkin's disease, rheumatoid arthritis, and systemic lupus erythematosus. Kasahara et al., Multiple Forms of Glycyl-prolyl Dipeptidyl-Aminopeptidase in Serum and Tissues, Japan. J. Clin. Chem., vol. 12, no. 1, pp. 89-93 (1983). It has been shown that DPP-IV inhibits the clotting of fibrin monomers. Mentlein et al., Dipeptidyl Peptidase IV Inhibits the Polymerization of Fibrin Monomers, Archives of Biochemistry and Biophysics, vol. 217, no. 2, pp. 748-750 (1982). DPP-IV has been located in the plasma membrane of rat and rabbit alveolar macrophages, hog endothelial cells, rat submandibular glands, human fibroblasts and rat hepatocytes, and serves as a surface marker for a subpopulation of human T-lymphocytes. Mentlein et al., Dipeptidyl Peptidase IV as a New Surface Marker for a Subpopulation of Human T-Lymphocytes, Cellular Immunology 89, pp. 11-19 (1984). DPP-IV reactions may also be of value in distinguishing and further characterizing T-lymphoproliferative disease states. Crockard et al., Dipeptidylaminopeptidase IV Activity in Normal and Leukemic T-Cell Subpopulations, [citation].

Various reagents and methods have been proposed in the prior art for the determination and study of peptidases. Many of these prior art procedures involve the hydrolysis of naphthylamides, particularly glycyl-DL-prolyl-beta-naphthylamide. Hopsu-Havu et al., A New Dipeptide Naphthylamidase Hydrolyzing Glycyl-Prolyl-beta-Naphthylamide, Histochemie pp. 197-201 (1966); Fujita et al., Serum Glycyl-proline p-Nitroanilidase Activity in Blood Cancers, Clinica Chimica Acta, 81, pp. 215-217 (1977); Heymann et al., Liver Dipeptidyl Aminopeptidase IV Hydrolyzes Substance P, FEBA Letters, vol. 91, no. 2, p. 360 (1978). The use of glycyl-proline-4-methoxy-2-naphthylamine (MNA) in assaying for peptidases, including DPP-IV, has been described in the literature. Lojda, Studies on Glycyl-Proline Naphythylamidase, Histochemistry 54, pp. 299-309 (1977); Lojda, Proteinases in Pathology. Usefulness of Histochemical Methods, J. Histochemistry and Cytochemistry, vol. 29, no. 3A, pp. 481-493 (1981); Gutschmidt et al., A Quantitative Histochemical Study of Dipeptidylpeptidase IV (DPP IV), Histochemistry 73, pp. 285-304 (1981) (using Fast Blue B as the coupling agent of choice); Lojda, The Importance of Protease Histochemistry in Pathology, Histochemical Journal

17, pp. 1063-1089 (1985) (using Fast Blue B).

## Summary of the Invention

Briefly describing one aspect of the present invention, there is provided a method for the colorimetric assaying for DPP-IV including combining an unknown with a substrate having a leaving group cleavable by DPP-IV, incubating the mixture to allow reaction of the DPP-IV, adding cinnamaldehyde to the unknown to combine with the leaving group to produce a color change, and evaluating the color change to determine the presence of DPP-IV.

It is an object of the present invention to provide a method for the colorimetric determination of DPP-IV in an unknown.

A further object of the present invention is to provide a colorimetric assay which is simple to perform and provides reliable, accurate results.

Another object of the present invention is to provide an assay procedure using cinnamaldehyde after hydrolysis to yield a detectable color change.

It is a further object of the present invention to provide a colorimetric assay for DPP-IV which involves a superior color change proportional to the amount of DPP-IV present in the unknown.

Further objects and advantages of the present invention will be apparent from the description of the preferred embodiment which follows.

## Brief Description of the Drawings

FIG. 1 is a diagram showing a method for preparing serial dilutions of the unknown sample for use in the assay method of the present invention.

FIG. 2 is a top, plan view of a test plate useful in performing the assay method of the present invention.

## Description of the Preferred Embodiment

In accordance with the present invention, there is provided a colorimetric assay for the determination of DPP-IV in an unknown sample. The method includes the combination of the unknown with a substrate and a color developing agent. The substrate includes a leaving group which is cleaved by the DPP-IV enzyme, and which thereafter combines with the color developer to produce a color change that is evaluated to determine the presence of DPP-IV.

The preferred embodiment of the assay procedure contemplates several components. In addition to the unknown, a buffer solution is useful in preparing the unknown for assay. Serial dilutions may be prepared by combining the unknown sample with the buffer solution. The progressively more dilute samples are then each assayed, and the differing colors developed for the respective dilutions are useful in evaluating the presence of DPP-IV. Various buffers suitable for the reaction components and unknown may be used, with a preferred buffer preparation being described in Example 1.

The serial dilutions of the unknown sample are prepared in accordance with standard technique. As shown in FIG. 1, the dilutions 10 may be prepared by filling six test tubes 11 each with 50 uL of the buffer solution 12. A 1:3 dilution is prepared by combining 25 uL of the unknown sample 13 with the first test tube 1. This combination is fully mixed, and 25 uL are then removed and added, as shown at 14, to the second test tube 2. This process is repeated through the six test tubes 1-6, yielding six sample dilutions.

The assay test is preferably performed in a test plate 15 having an array of individual test wells 26 for each assay. As shown in FIG. 2, a preferred test plate 15 includes three rows 16-18 of test wells arranged in seven columns 19-25. For a given assay, a predetermined amount of each of the six dilutions 1-6 of the sample is placed into the first six test wells in a selected row. The seventh test well may be used as a control, such as by delivering a predetermined amount of the buffer solution into the test well. The test plate of FIG. 2 may also be conveniently used to assay two different samples in the first two rows, and a control such as normal pooled sera in the third row.

The assay method of the present invention uses a substrate which includes a leaving group that is cleavable by DPP-IV and which combines with cinnamaldehyde to form a color change. The substrate may comprise any of a variety of conventional components, namely amino acid containing, water dispersible substrates, including a leaving group produced as a result of cleavage by DPP-IV. The leaving group is selected from many which form a suitable color change upon combination with cinnamaldehyde.

The reagents and tests preferably use as a substrate the dipeptide L-Alanyl-L-Proline attached to a leaving group through its amine by forming an arylamide bond. The substrate for the test may be impregnated into special paper disks 27 and placed into the test well for performing the assay. The enzyme cleaves the arylamide bond, liberating the leaving group in quantity directly proportional to the amount of active enzyme present. The substrate may comprise a variety of peptides corresponding to enzymes to be assayed. The peptides may be any appropriate length arrangement, usually up to eight amino acids. Specific examples are described in United States Patent No. 3,862,011, issued to Smith on January 21, 1975, and that disclosure is hereby incorporated by reference. Such peptides may include for example;: Leucyl-; Lysyl-alanyl-; Prolyl-arginyl-; Glycyl-prolyl-; and Arginyl-arginyl-.

The present invention pertains to the use of cinnamaldehyde (p-dimethylcinnamaldehyde) as the color developing reagent in the colorimetric assay for DPP-IV. The assay may be performed for DPP-IV in a variety of sample specimens, such as T-lymphocyte subsets, blood plasma, blood serum and other cell exudates or fluids. The sample specimen is combined with the substrate, and the DPP-IV cleaves the arylamide bond of the substrate.

The liberated leaving group 4-methoxy-2-naphthylamine, for example, couples with the cinnamaldehyde to produce a magenta color within a maximum of ten minutes, indicating a positive reaction. In this embodiment, a magenta to pale pink color identifies a positive reaction, whereas a pale yellow demonstrates a negative reaction. The invention utilizes a Schiff-base reaction (in acid), and many different leaving groups provide color change with cinnamaldehyde, and therefore are useful with the present invention. Examples of leaving groups coupled to cinnamaldehyde are as follows, with the color on the substrate paper as indicated:

1. 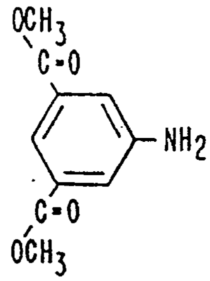

pink

AIE 5-Aminoisophthalic acid dimethyl ester

2. 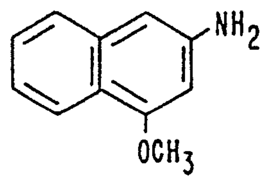

magenta

MNA 4-methoxy-2-naphthyl amine

3. 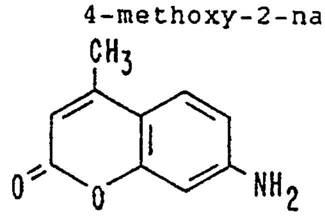

magenta to violet

AMC 7-amino-4-methyl coumarin

4. 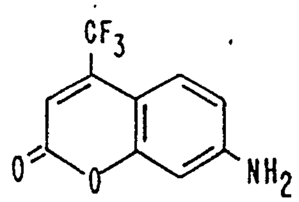

purple

AFC 7-amino-4-trifluoromethyl coumarin

5. 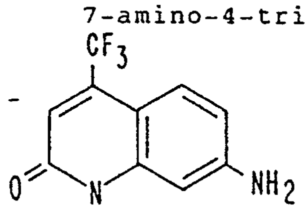

purple

AFQ 7-amino-4-trifluoromethyl

6. 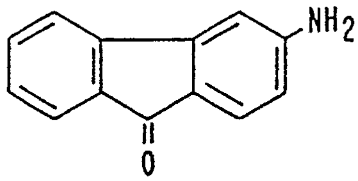

blue

3-amino-9-fluorenone

7. 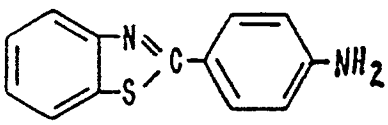

green

APB 2-(4-amino phenyl)-6-methyl benzthiazale

8. 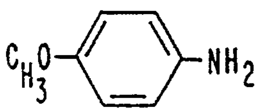

pink-peach

AMB panesoli (4-methoxyamiline)

9. 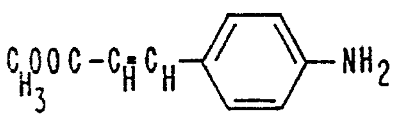

magenta

MAC methylcinnamic ester

10. 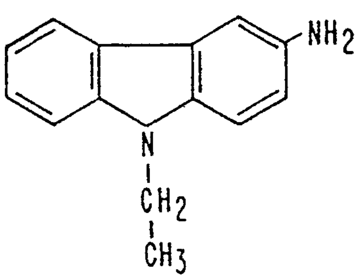

violet

AEC 3 amino-9-ethylcarbozole

The colorimetric test is preferably performed in a white test plate which enhances visual color definition. The end-point test can be visually read or the reaction product can be quantitated with a simple spectrophotometer.

In the test system of the preferred embodiment, the assay test is performed upon 1:3 serial dilutions of the sample specimen. The substrate for the assay is preferably impregnated into special paper disks. A reaction disk and the buffer solution is then combined in a test well, to which is added the sample specimen.

Controls may be employed to assure the reproducibility of the assay results. One type of control is the use of a known control, such as normal pooled sera, which is assayed in the same manner as the sample specimen. Also, a separate control may be employed by combining the cinnamaldehyde reagent with the buffer solution and a reaction disk in a test well. A positive reaction for the sample specimen is indicated when any of the test wells displays the indicative color change. A negative reaction will show no change in color from the buffer substrate control.

The colorimetric assay of the present invention has been found to provide excellent results for determination of DPP-IV. Certain limitations will apply to the test as for all such assays. For example, results obtained from clinically healthy individuals may be influenced by diet, sex, age, physical activity, pregnancy, and environmental factors. Medications which impart a certain color in bodily fluids may give variable results in the colorimetric detection of the test. Red blood cells in the sample may cause a change from the expected color detection. Proper separation technique should eliminate excessive red blood cells present in the sample. Also, excessive vortexing may degrade the enzyme and thus give variable results. Since DPP-IV activity in plasma, sera, or mononuclear cells will decline over time, even if stored frozen at -70 °C., assays should be conducted in a timely fashion. Tissue media containing fetal calf serum may also give variable results, and storage of cells in fetal calf serum or using fetal calf serum in mononuclear cell separation should be avoided.

Preferred embodiments of the present invention are illustrated in the following examples.

## Example 1

Preparation of the components for the final reaction mixtures were prepared as follows.

A buffer solution was produced by mixing the following ingredients: 6.05 g. Trizma buffer (dry), 0.05 g. sodium azide, 5.0 ml. Triton X-100, 500 ml. distilled water (reagent grade), and a sufficient quantity of 1 N. hydrochloric acid to adjust the solution pH to 7.8 plus or minus 0.1.

A stock solution of the substrate Alanyl-Prolyl-MNA was prepared by adding 7.55 g. of dry Alanyl-Prolyl-MNA to 1.0 ml. of N,n dimethylformamide to form a 20 mM stock solution. Reaction disks impregnated with the Alanyl-Prolyl-MNA were prepared as follows.

Two separate substrate preparations were made, one for use in a visual evaluation of the test results and the other for an automated colorimetric method. For the visual method, 125 uL of the stock Alanyl-Prolyl-MNA solution was added to 10.0 ml. distilled water to make a working solution. Number 3 qualitative-grade filter paper was saturated with the working solution and allowed to dry. The dried, impregnated paper was then cut into 4.0 mm. disks. One disk was used in each reaction well in a test tray. To each test well was added 25 uL of a diluted sample, and 10 uL of p-dimethylcinnamaldehyde, for a final Alanyl-Prolyl-MNA concentration of 0.106 mM.

For use in an automated colorimetric method, the stock Alanyl-Prolyl-MNA solution was diluted 1:20 in the prepared buffer solution. 50 uL of the resulting solution was added to each reaction well of a test plate, and to that was added 50 uL of the diluted sample, and 50 uL of the p-dimethylcinnamaldehyde, for a final Alanyl-Prolyl-MNA concentration of 0.333 mM.

Third, a p-dimethylcinnamaldehyde solution was prepared by adding 0.1 g. p-dimethylcinnamaldehyde (dry) to 9.5 ml. ethanol (95%) and 20 ml. concentrated hydrochloric acid (12 N).

Appropriate care for the prepared reagents should be taken. For example, the present invention may be used as a kit for the quantitative testing of the DPP-IV enzyme in various specimens. Such test kits should preferably be stored and maintained in a fashion to assure the integrity of the reagents. Storage of such materials is recommended at a temperature of 2-8 °C. The reaction disks should be kept dry and shielded from bright light. All unused reaction disks should be stored in plastic vials with a dessicant.

## Example 2

The visual colorimetric assay for the DPP-IV enzyme was performed using a test plate configured to accomodate testing of two samples and a control as shown in FIG. 2. The test plate had an array of three rows of seven columns of test wells.

Using forceps, one reaction disk was placed into each well of the test plate.

For each specimen to be assayed, six (6) serial 1:3 dilutions in the specimen were prepared in accordance with the diagram of FIG. 1. Beginning with six (6) tubes including 50 uL each of the buffer solution, a 25 uL portion of the test sample was added to the first tube. From the resulting 75 uL sample dilution, 25 uL was taken and added to the second tube. This process was repeated thereby providing six (6) samples at progressive 1:3 dilutions. For a given specimen, 25 uL of each of the sample dilutions (6 total) were added to the respective wells on the test plate. (Pipette tips should be changed between dilutions, or this procedure should be started with the last, weakest dilution and progressed to the stronger dilutions.) 25 uL of the buffer solution were added to the buffer substrate control well in the seventh column of the test wells.

5 ml. of tap water were added to an incubation tray and the test plate was placed into the tray. The lid of the incubation tray was put in place, and the test plate was incubated for 30 minutes at 37-42° C. The tray and test plate were removed from the incubator and 10 uL of the p-dimethylcinnamaldehyde solution was added to each well.

The visual colorimetric reaction mixture therefore comprised 0.106 mM Alanyl-Prolyl-MNA, 5.5 mM p-dimethylcinnamaldehyde, 16.66 uL to 24.97 uL of the buffer solution, 8.34 uL to 0.03 uL of the sample specimen, 6.78 uL hydrochloric acid (conc.), and 3.22 uL ethanol (95%), for a total volume of 35 uL.

The test was then ready for interpretation, with reactions preferably being read within 10 minutes.

The test plate also provided for assay of two samples and a control. The third row was used as a positive control by assaying with normal pooled sera (e.g., Monitrol I, American Dade, Division of American Hospital Supply, Inc.) to insure reproducibility. Good results with the control test were obtained.

## Example 3

Mononuclear cells (MNC's) were collected for assay. 5 ml. of blood were collected by venipuncture in EDTA tubes. 4 ml. of RPMI minus glutamine medium (room temperature) was added to 4 ml. of the whole EDTA blood. The mixture was capped and inverted three (3) times and stored at room temperature. The mononuclear cells were separated within 24 hours of blood collection.

Separation of the mononuclear cells was accomplished as follows. Into a 5 ml. centrifuge tube was placed 4 ml. Ficoll-Hypaque (room temperature). Using a transfer pipette, 8 ml. of 1:1 (whole EDTA blood RPMI) mixture was carefully overlaid onto the Ficoll-Hypaque, and the tube was capped. The tube was then centrifuged at 400 G. for 30 minutes at 25° C. (plus or minus 5° C.). The MNC's formed a visible interface between the plasma and the Ficoll-Hypaque. The top plasma-RPMI layer was aspirated to within 2 ml. of the MNC layer and discarded. The MNC layer was collected to 2 ml. below the opaque interface, using a transfer pipette, and placed into a new 15 ml. tube. RPMI was then added to the 15 ml. mark, the tube capped and inverted three (3) times. The tube was then centrifuged at 250 G. for 10 minutes at 25° C. (plus or minus 5° C). Any excess RPMI was carefully aspirated from the cell plug with a pipette. 150 uL of the buffer solution was added to the plug and mixed thoroughly, avoiding vortexing, and this mixture formed the base for assay purposes.

Serial 1:3 dilutions of the base were prepared using the buffer solution, and the sample dilutions were used in performing the enzyme test of Example 2, yielding excellent results.

## Example 4

DPP-IV testing was also performed upon mononuclear cells collected in accordance with the procedure of Example 3, but which were frozen at -70° C. for less than two weeks. In this procedure, the base solution of mononuclear cells was placed into a cryogenic vial and capped. The vial was frozen completely and then allowed to thaw at room temperature. This freeze-thaw procedure was followed two times. The freeze-thaw process was performed either in a -70° C. freezer or an isopropanol-dry-ice bath. Appropriate dilutions of the base material were then prepared in accordance with the outline of FIG. 1, and assays were performed in the same manner as Example 2 with excellent results.

## Example 5

A plasma sample was obtained for assay in accordance with the following procedure. Whole EDTA blood obtained by the procedure of Example 3 was centrifuged at 250 G. for 10 minutes at 25° C. (plus or minus 5° C). The plasma was aspirated into a separate container. The plasma was stored at -70° C. if not tested immediately. Appropriate dilutions of the plasma were prepared by the addition of buffer solution in accordance with the procedure of Example 2. The dilutions were used

for conducting the colorimetric assay of Example 2 and yielded excellent results.

Example 6

For use in a colorimetric method, the stock Alanyl-Prolyl-MNA solution was diluted 1:20 in the prepared butter solution. 50 uL of the resulting solution was added to each test cuvette and to a blank cuvette of a colorimeter capable of measuring optical density at a wavelength of 540 nM. To each test cuvette was added 50 uL of a sample of peripheral white blood cells separated from anticoagulated blood and lysed in a 1% solution of Triton X-100 detergent in 0.1 M Trizma buffer (pH 7.8).

Into the blank cuvette, 50 uL of the 1% solution of Trizma buffer was added.

The cuvettes were incubated at 37-42° C. in high humidity for 30 minutes. The cuvettes were then removed from the incubator and 50 uL of p-dimethylcinnamaldehyde solution was added to each well.

the colorimetric reaction mixture therefore comprised 0.333 mM Alanyl-Propyl-MNA, 15 mM p-dimethylcinnamaldehyde, 16.7 uL of sample specimen, 80.8 uL 1% Triton X-100 solution, 8.3 uL of hydrochloric acid (conc.), 41.7 uL ethanol (95%), and 2.5 uL of N,N dimethylformamide, for a total volume of 150 uL.

The test was then ready for interpretation, with reactions read at a wavelength of 540 nM. Good results, expressed in International Units, were obtained.

Example 7

Other body fluids were collected and prepared for assay in accordance with the prior procedures. Subject to the limitations previously described, the assay procedure yielded good results for body fluids, including T-lymphocyte subsets, blood serum and other cellular exudates/fluids.

Example 8

The foregoing tests were conducted by use of an automated colorimetric assay device known as the "Multistat". As indicated in Example 1, 50 uL of the Alanyl-Prolyl-MNA test solution for automated assays was added into each test cuvette of a Multistat wheel and into the reference cuvette. 50 uL of each of the 1:3 sample dilutions were also loaded into the test cuvettes, and a 1:3 dilution of standard was also loaded into one test cuvette. 50 uL of the buffer solution was added to the reference cuvette. The Multistat wheel was incubated at 37° C. for 30 minutes, and to each test cuvette and to the reference cuvette was added 50 uL of the cinnamaldehyde reagent. The wheel was loaded immediately into the Multistat, and the automatic assay program was initiated. For the Multistat device, the following parameters were used: delay time 3 seconds, interval time 3 seconds, intervals 3, filter code 6 (550 nM), and start mode 1. The results were reported in IU/L of whole blood and excellent results were obtained.

Example 9

The foregoing tests were repeated with various peptides and leaving groups. The peptides include Leucyl-; Lysyl-alanyl-; Prolyl-arginyl-; Glycyl-prolyl-; and Arginyl-arginyl-. The leaving groups include 5-aminoisophthalic acid dimethyl ester; 4-methoxy-2-naphthylamine; 7-amino-4-methyl coumarin; 7-amino-4-trifluoromethyl coumarin; 7-amino-4-trifluoromethyl; 3-amino-9-fluorenone; 2-(4-aminophenyl)-6-methylbenzthiazale; panesoli (4-methoxyaniline); methylcinnamic ester; and, 3-amino-9-ethylcarbozole. Good results are obtained.

While the invention has been illustrated and described in detail in the drawings and foregoing description, the same is to be considered as illustrative and not restrictive in character, it being understood that only the preferred embodiment has been shown and described and that all changes and modifications that come within the spirit of the invention are desired to be protected.

## Claims

1. A method for the colorimetric assaying for DPP-IV in an unknown, which method comprises the steps of:

a. combining with the unknown a substrate which includes a leaving group cleavable by DPP-IV, the leaving group being combinable with cinnamaldehyde to produce a detectable color change;

b. incubating the mixture of step a. to allow reaction of the DPP-IV with the substrate;

c. before or after the incubating of step b., adding cinnamaldehyde to the unknown, the cinnamaldehyde combining with the cleaved leaving group to produce a color change; and

d. evaluating the color change to determine the presence of DPP-IV.

2. The method of claim 1 in which the leaving group is cleaved in an amount directly proportional to the amount of DPP-IV present in the unknown.

3. The method of claim 1 in which the substrate includes L-Alanyl-L-Proline coupled with the leaving group.

4. The method of claim 3 in which the L-Alanyl-L-Proline is coupled with the leaving group through the amine, forming an arylamide bond.

5. The method of claim 1 in which the leaving group is 4-Methoxy-2-Naphthylamine.

6. The method of claim 5 in which the substrate includes L-Alanyl-L-Proline coupled with the 4-Methoxy-2-Naphthylamine.

7. The method of claim 6 in which the substrate is impregnated on a carrier.

8. The method of claim 1 in which the substrate includes a peptide constituent selected from the group consisting of: Leucyl-; Lysyl-alanyl-; Prolyl-arginyl-; Glycyl-prolyl; and Arginyl-arginyl-.

9. The method of claim 1 in which the leaving group is selected from the group consisting of: 5-aminoisophthalic acid dimethyl ester; 4-methoxy-2-naphthylamine; 7-amino-4-methyl coumarin; 7-amino-4-trifluoromethyl coumarin; 7-amino-4-trifluoromethyl; 3-amino-9-fluorenone; 2-(4-aminophenyl)-6-methylbenzthiazale; panesoli (4-methoxyaniline); methylcinnamic ester; and, 3-amino-9-ethylcarbozole.

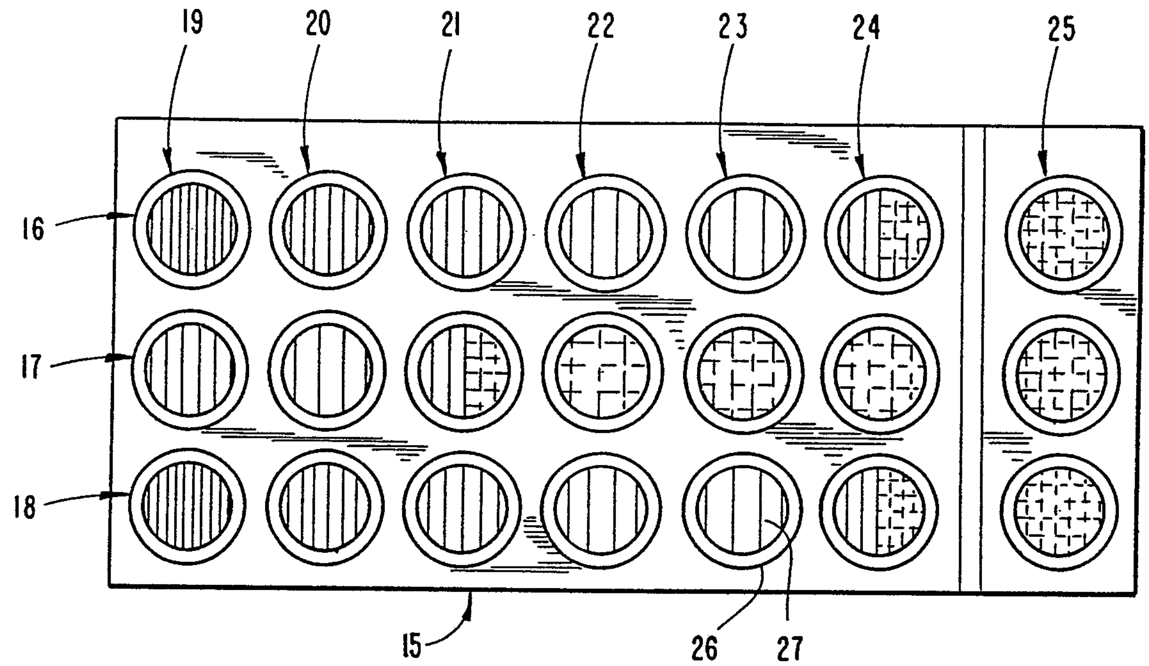
19
20
21
22
23
24
25
16
17
18
15
26
27

Fig.2

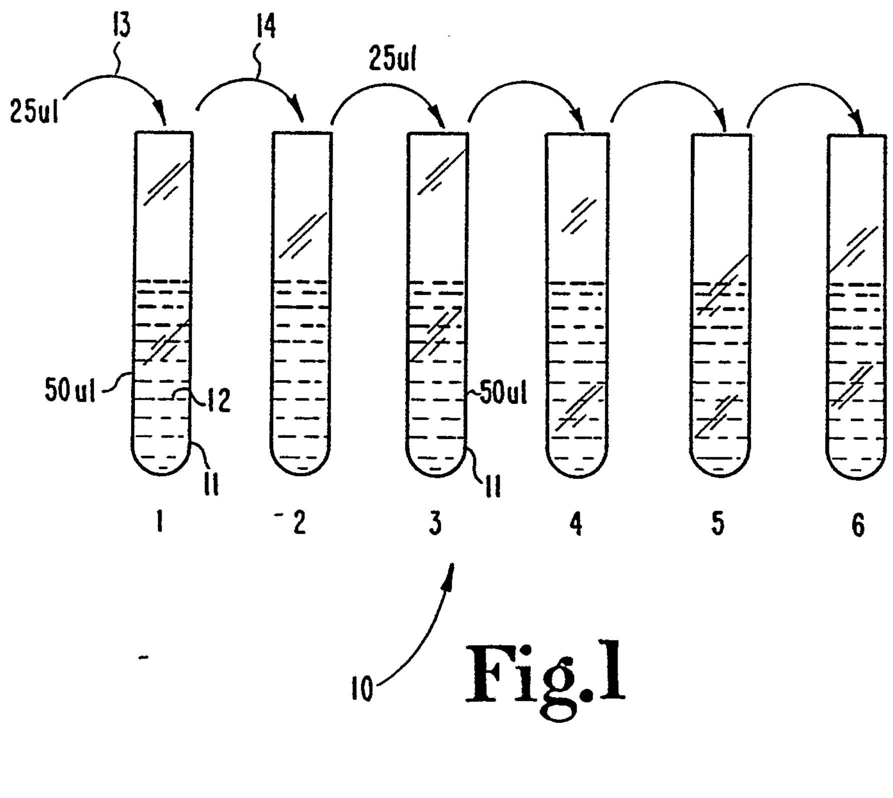
13
14
25ul
25ul
50ul
12
11
50ul
11
1
2
3
4
5
6
10

Fig.1